# EUROPEAN PATENT APPLICATION

(11) **EP 4 295 856 A1**
(43) Date of publication of application: **27.12.2023**
(21) Application number: 21926606.1
(22) Date of filing: 19.02.2021
(51) Int. Cl.: A61K 36/185, A61P 17/00, A61P 37/04, A61P 43/00, A23L 33/105, A61K 8/9789, A61Q 19/08

(54) **IMMUNE CELL ACTIVATOR, ANTI-AGING AGENT, METHOD FOR ACTIVATING IMMUNE CELLS, AND METHOD FOR PREVENTING OR PROTECTING AGAINST AGING**

(71) Applicant: Shiseido Company, Ltd., Chuo-ku Tokyo 104-0061 (JP)
(72) Inventor: HOSOI, Junichi, Tokyo 104-0061 (JP); HASEGAWA, Tatsuya, Tokyo 104-0061 (JP)
(74) Representative: Cabinet Beau de Loménie
(86) International application number: PCT/JP2021/006476
(87) International publication number: WO 2022/176174

(57) **Abstract**

The present invention provides an immune cell activator containing hibiscus or a fermented extract thereof as an active ingredient. The present invention also provides an anti-aging agent characterized in that said anti-aging agent contains hibiscus or a fermented extract thereof as an active ingredient and eliminates senescent cells by activating immune cells. The present invention additionally provides a method for activating immune cells in a subject in need thereof, wherein the method includes administering hibiscus or a fermented extract thereof to the subject and activating the immune cells thereof. In addition, the present invention provides a method for preventing or protecting against aging in a subject in need thereof, wherein the method includes administering hibiscus or a fermented extract thereof to the subject and eliminating senescent cells by activating immune cells.

## Description

### FIELD

The present invention relates to an immune cell activator, to an anti-aging agent, to a method for activating immune cells and to a method for preventing or protecting against aging.

### BACKGROUND

Senescent cells are known to increase in the body as aging progresses (NPL 1). Since it is known that senescent cells are eliminated by immune cells (such as NK cells, monocytes/macrophages and T cells) (NPL 2), and it has been reported that senescent cell-eliminating immune cells decrease with aging (NPL 3), activation of immune cells is considered important for eliminating the senescent cells that increase with aging.

The activation markers NKp30 and NKp46 on NK cells, one type of immune cell that eliminates senescent cells, are known to be expressed in lower amounts with aging (NPL 3). The marker NKp46 is known to be involved in immune synapse formation between NK cells and their target cells (NPL 4), suggesting that NKp46 expression is extremely important for activation of NK cells.

Fermentation products of hibiscus or extract thereof are known to promote synthesis of hyaluronic acid by epidermal cells, and skin external compositions for improvement of skin wrinkles and sagging are known that include such components (PTL 1). As explained below, however, the present inventors are first to have demonstrated the immune cell-activating effects of fermentation products of hibiscus or extract thereof, which have not been reported on to date.

### [CITATION LIST]

### [PATENT LITERATURE]

[PTL 1] Japanese Unexamined Patent Publication No. 2019-34899

### [NON PATENT LITERATURE]

[NPL 1] van Deursen JM., Nature., 2014, 509:439-446
[NPL 2] Sagiv A., and Krizhanovsky V., Biogerontology. 2013 Dec; 14(6):617-628.
[NPL 3] Almeida-Oliveira A., et al., Hum Immunol. 2011 Apr; 72(4):319-329.
[NPL 4] Hadad U., et al., J Immunol May 1, 2014, 192 (1 Supplement) 121.13

### SUMMARY

### [TECHNICAL PROBLEM]

It is an object of the present invention to provide a novel immune cell activator that activates immune cells, and a method for activating immune cells using it. It is another object of the invention to provide a novel anti-aging agent that eliminates senescent cells by activation of immune cells, as well as a method for preventing or protecting against aging using it.

### [SOLUTION TO PROBLEM]

As a result of extensive research, the present inventors have been the first to find that fermentation products of hibiscus or extract thereof have an effect of activating immune cells, and the invention has been completed upon this finding. Specifically, the present invention encompasses the following inventions.

[1] An immune cell activator comprising a fermentation product of hibiscus or extract thereof as an active ingredient.
[2] The immune cell activator according to [1] above, wherein the immune cells are NK cells.
[3] The immune cell activator according to [1] or [2] above, wherein senescent cells are eliminated by activation of the immune cells.
[4] The immune cell activator according to any one of [1] to [3] above, wherein the fermentation product of hibiscus or extract thereof is obtained by lactic acid bacteria fermentation of the hibiscus or extract thereof.
[5] An anti-aging agent comprising a fermentation product of hibiscus or extract thereof as an active ingredient, and that eliminates senescent cells by activation of immune cells.
[6] The anti-aging agent according to [5] above, wherein the immune cells are NK cells.
[7] The anti-aging agent according to [5] or [6] above, wherein the fermentation product of hibiscus or extract thereof is obtained by lactic acid bacteria fermentation of the hibiscus or extract thereof.
[8] A method for activating immune cells for a subject in need thereof, comprising:
   applying a fermentation product of hibiscus or extract thereof to the subject to activate the immune cells.
[9] The method according to [8] above, wherein the immune cells are NK cells.
[10] The method according to [8] or [9] above, wherein senescent cells are eliminated by activation of the immune cells.
[11] The method according to any one of [8] to [10] above, wherein the fermentation product of hibiscus or extract thereof is obtained by lactic acid bacteria fermentation of the hibiscus or extract thereof.
[12] A method for preventing or protecting against aging for a subject in need thereof, comprising:
   applying a fermentation product of hibiscus or extract thereof to the subject to eliminate senescent cells by activation of immune cells.
[13] The method according to [12] above, wherein the immune cells are NK cells.
[14] The method according to [12] or [13] above, wherein the fermentation product of hibiscus or extract thereof is obtained by lactic acid bacteria fermentation of hibiscus flower extract.

### [ADVANTAGEOUS EFFECTS OF INVENTION]

According to the invention, it is possible to activate immune cells, thereby eliminating senescent cells.

### BRIEF DESCRIPTION OF DRAWINGS

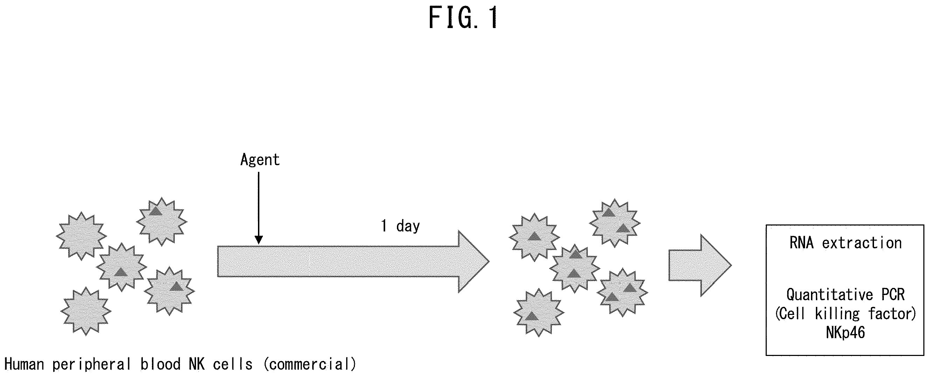

Fig. 1 is a diagram schematically illustrating the screening method used for selection of an immune cell activator or anti-aging agent of the invention.
Fig. 2 is a graph showing activation of NK cells by an immune cell activator or anti-aging agent of the invention. Shown are NKp46 mRNA expression levels in NK cells with addition of hibiscus fermentation product at different concentrations (v/v%), as standardized values against RPS9 mRNA expression level as an endogenous control.

### DESCRIPTION OF EMBODIMENTS

Embodiments for carrying out the invention will be described in detail below, with the understanding that the technical scope of the invention is not limited only to these embodiments. The descriptions of the embodiments for carrying out the invention are mutually applicable for explanation of the immune cell activator, anti-aging agent, method for activating immune cells and method for preventing or protecting against aging, unless otherwise specified. The prior art documents cited herein are incorporated in their entirety by reference throughout the present specification.

### <Immune cell activator and anti-aging agent>

According to one embodiment, the invention provides an immune cell activator comprising a fermentation product of hibiscus or extract thereof as an active ingredient. According to another embodiment, the invention provides an anti-aging agent comprising a fermentation product of hibiscus or extract thereof as an active ingredient, and that eliminates senescent cells by activation of immune cells.

Throughout the present specification, "hibiscus" refers to a plant belonging to Malvaceae Hibiscus, examples of which include roselle (*Hibiscus sabdariffa* L.), rose of Sharon (*Hibiscus syriacus*), cotton rose (*Hibiscus mutabills*), scarlet hibiscus (*Hibiscus coccineus*), sea hibiscus (*Hibiscus tiliaceus*) and Japanese lantern (*Hibiscus schizopetalus*). The "hibiscus or extract thereof" for the purpose of the invention may be from any part such as the entire plant, leaves, flowers, stems, roots or grains. When flowers are used, the flowering period and size are not particularly restricted, and they may include the petals or calyx, or the entirety.

The method of preparing the "hibiscus extract" to be used for the invention may be a publicly known method, and it may be prepared with reference to Japanese Unexamined Patent Publication No. 2019-34899, for example. The following is an example of a procedure for preparing a "hibiscus extract".

The hibiscus extraction site is first washed to remove contaminants if necessary and used either directly or after drying, with chopping or pulverizing as necessary, and then contacted with an extraction solvent for extraction. The extraction may be carried out by contact with an extraction solvent by a common method such as immersion, or supercritical extraction or steam distillation may be employed instead.

Extraction solvents include water; lower alcohols such as methanol, ethanol and propanol and higher alcohols such as oleyl alcohol, stearyl alcohol and octyldodecanol; polyhydric alcohols such as ethylene glycol, 1,3-propanediol, 1,3-butylene glycol and glycerin; esters such as ethyl acetate, butyl acetate, methyl propionate and glyceryl trioctanoate; ketones such as acetone and methyl ethyl ketone; ethers such as ethyl ether, isopropyl and ether; and hydrocarbon-based solvents such as *n*-hexane, toluene and chloroform, any of which may be used alone or in mixtures of two or more.

The mixing ratio when using a mixed solvent is preferably in the range of 1:1 to 25:1 (volume ratio, same hereunder) for a mixed solvent of water and ethyl alcohol, in the range of 1:1 to 15:1 for a mixed solvent of water and glycerin, or in the range of 1:1 to 15:1 for a mixed solvent of water and 1,3-propanediol or 1,3-butylene glycol.

The pH used when preparing the extract is not particularly restricted but is generally preferred to be in the range of pH 3 to 9. If necessary, an alkaline adjustor such as sodium hydroxide, sodium carbonate or potassium hydroxide or an acidic adjustor such as citric acid, hydrochloric acid, phosphoric acid or sulfuric acid may be added to the extraction solvent for adjustment to the desired pH.

The extraction conditions such as the extraction temperature and extraction time are not limited as they will differ depending on the type of solvent used and the pH, but when the solvent is water, 1,3-butylene glycol or a mixture of water and 1,3-butylene glycol, for example, the extraction temperature may be in the range of 0°C to 90°C and the extraction time may be 0.5 hour to 7 days.

The extraction site may also be subjected to hydrolysis if necessary, before the extraction treatment or simultaneously with the extraction treatment. This can improve the skin irritation, effectiveness or storage stability of the extract to allow the extract to be more effectively utilized.

For the purpose of the present invention, a "fermentation product of hibiscus or extract thereof" is one obtained by publicly known fermentation of "hibiscus or extract thereof" using a microorganism (for example, lactic acid bacteria, yeast, *Aspergillus oryzae* or *Bacillus subtilis*), and it can be prepared with reference to Japanese Unexamined Patent Publication No. 2019-34899, for example. The "fermentation product of hibiscus or extract thereof" to be used for the invention is preferably one obtained by lactic acid bacteria fermentation, and for example, the commercially available product HIBIZELLE^{R} (also known as "Hibiscus Flower Fermentation Extract") (Technoble, Japan), prepared by fermentation of hibiscus extract using lactic acid bacteria, may be used for the invention.

Immune cells can be activated by applying the activator of the invention. As used herein, "immune cells" are cells that perform the role in the "immune system" in the body, and they include NK cells, T cells, B cells, monocytes/macrophages and dendritic cells, for example. The immune cells to be activated according to the invention are preferably NK cells.

NK cells (natural killer cells) are a type of cytotoxic lymphocyte functioning as a major factor in natural immunity. NK cells do not express T cell receptors (TCR), CD3 or surface immunoglobulin (Ig) B cell receptors, but in humans they are generally CD16 (FcγRIII)-positive and CD56-positive. NK cells are cytotoxic, and the small granules in their cytoplasm contain specialized proteins such as proteases known as perforins and granzymes. When released in close proximity to cells targeted for killing, perforins form holes in the cell membranes of the target cells, allowing infiltration of granzymes and related molecules and inducing apoptosis.

NK cells are normally activated in response to interferon or macrophage-secreted cytokines. The cytotoxicity of NK cells is highly regulated by two types of surface receptors, known as "activating receptors" and "inhibitory receptors". It is known that NK cells perform an important role in host rejection of tumors, while also playing a role in killing of virus-infected cells.

Activated immune cells such as NK cells and T cells are also known to play a role in elimination of senescent cells (Sagiv A., and Krizhanovsky V., Biogerontology. 2013 Dec; 14(6):617-628.). The aforementioned activation markers on NK cells, NKp30 and NKp46, are known to have decreased expression levels with aging (Almeida-Oliveira A., et al., Hum Immunol. 2011 Apr; 72(4):319-329.). Based on this knowledge, the present inventors searched for substances that increase expression levels of NK cell activation markers, and have completed this invention upon finding that fermentation products of hibiscus or extract thereof exhibit such an effect.

As used herein, "senescent cells" refers to cells exhibiting increased expression levels of aging markers compared to normal cells. Aging markers include senescence-associated β-galactosidase, P53, P16^{INK4a} and P21^{CIP1}. Senescent cells are characterized by irreversible growth arrest in the G1 phase, and are known to form in association with suppression of genes that promote progression of the cell cycle and increased expression of p53, p16^{INK4a} and p21^{CIP1} that inhibit the cell cycle.

Senescent cells may result from replicative cell senescence, premature cell senescence or therapy-induced cell senescence. Senescent cells formed by replicative cell senescence may have gone through multiple cell divisions, such as 40 or more, 50 or more, 60 or more, 70 or more or 80 or more cell divisions. Senescent cells formed by premature cell senescence may be induced by factors that include but are not limited to ultraviolet light, reactive oxygen species, environmental toxins, smoking, ionizing radiation, chromatin structure distortion, excessive mitogen signaling and carcinogenic mutation. According to another embodiment, premature cell senescence can be induced by ionizing radiation. According to yet another specific embodiment, premature cell senescence can be induced by ectopic transfection using Ras protein. Senescent cells resulting from therapy-induced cell senescence can be induced by radiotherapy, chemotherapy or DNA damage therapy.

Senescent cells that can be eliminated by use of the invention may generally be eukaryotic cells. Examples of senescent cells include, but are not limited to, mammary epithelial cells, keratinocytes, myocardial cells, chondrocytes, endothelial cells (large artery), endothelial cells (microvessels), epithelial cells, fibroblasts, dermal papilla cells, hepatocytes, melanocytes, osteoblasts, adipose precursor cells, immune system cells, skeletal muscle cells, smooth muscle cells, adipocytes, neurons, glial cells, contractile cells, exocrine epithelial cells, extracellular matrix cells, hormone secretion cells, cornified epithelial cells, pancreatic islet cells, lens cells, mesenchymal stem cells, pancreatic adenocarcinoma cells, Paneth cells of the small intestine, hematopoietic cells, nervous system cells, supporting cells of sensory organs and peripheral neurons, and moist stratified barrier epithelial cells.

The number of senescent cells in various organs and tissues generally rises with increasing age. Accumulation of senescent cells can further promote aging and deterioration of age-related diseases. For example, accumulation of senescent cell in aged tissues may contribute to aged-related tissue dysfunction, decreased regenerative power and disease. According to one embodiment, aged tissue with accumulated senescent cells lack the ability to respond to the stress required for growth, thereby resulting in the reduced health seen with aging. According to one embodiment, therefore, application of the invention causes elimination of senescent cells by immune cells, and can thus prevent or protect against aging.

According to one embodiment, the invention may provide a composition comprising an immune cell activator or an anti-aging agent. The composition of the invention may be in any of a variety of forms such as powder, liquid, solid, granular, pasty, gel-like, latex, cream, sheet, spray or foam.

The content of the immune cell activator or anti-aging agent in the composition of the invention is not particularly restricted, and may include an amount effective for activation of immune cells.

The composition of the invention may optionally contain various additives if necessary. Excipients or the like may be added as additives.

Excipients may be any that are commonly used for the desired form, and examples include starches such as wheat starch, rice starch, corn starch, potato starch, dextrin and cyclodextrin, crystalline celluloses, saccharides such as lactose, glucose, sugar, reduced maltose, rice jelly, fructooligosaccharides and emulsified oligosaccharides, and sugar alcohols such as sorbitol, erythritol, xylitol, lactitol and mannitol. Any of these excipients may be used alone or in combinations of two or more.

The composition of the invention may also employ other components appropriately selected from among publicly known components such as coloring agents, preservatives, thickeners, binders, disintegrators, dispersing agents, stabilizers, gelling agents, antioxidants, surfactants, preservatives, pH regulators, oils, powders, colorants, water, alcohols, thickeners, chelating agents, silicones, antioxidants, ultraviolet absorbers, humectants, flavors, drug components, antiseptic agents, pH regulators or neutralizers, as necessary.

### <Method for activating immune cells and method for preventing or protecting against aging>

According to one embodiment, the invention provides a method for activating immune cells in a subject in need thereof, comprising:
applying a fermentation product of hibiscus or extract thereof to the subject to activate the immune cells.

According to another embodiment, the invention provides a method for preventing or protecting against aging for a subject in need thereof, comprising:
applying a fermentation product of hibiscus or extract thereof to the subject to eliminate senescent cells by activation of immune cells.

As used herein, the term "subject" refers to a mammal such as a human, and is preferably a human subject. The method for application of the fermentation product of hibiscus or extract thereof may be local administration (skin, inhalation, enema, eye drop, ear drop, nasal or intravaginal), enteral administration (oral, tubal or intravenous) or parenteral administration (intravenous, transarterial, transdermal or intramuscular injection).

### EXAMPLES

The present invention will now be explained in greater detail by Examples, with the understanding that the invention is not limited in any way by the Examples.

### 1. Materials

The following materials were used in the Examples.
· Primary cultured human NK cells (Promocell)
· Medium: RPMI 1640 medium with addition of 20% fetal calf serum and essential amino acids/non-essential amino acids (Nacalai Tesque, Inc.)
· Hibiscus flower fermentation extract (HIBIZELLE^{R}, Catalog No. 05694, Technoble, Japan)

### 2. Experiment method

The commercial (Promocell) Primary cultured human NK cells were cultured in the RPMI 1640 medium containing added 20% fetal calf serum and essential amino acids/non-essential amino acids (Nacalai Tesque, Inc.) (37°C, 100% humidity, 5% CO₂).

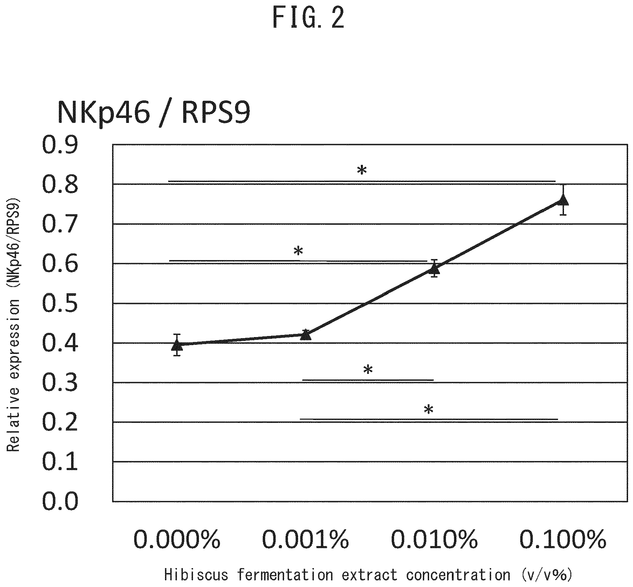

NK cells (4 × 10⁵) suspended in 1 ml of medium were inoculated into a 24-well plate and PBS-diluted Hibiscus flower fermentation extract (05694) was added in the concentration listed in Fig. 2. After culturing for 24 hours, an RNeasy Plus (Qiagen) was used to extract the RNA from the collected cells, and GoScript transcriptase (Promega) was used for cDNA synthesis. Semi-quantitative PCR of the NKp46 gene and the ribosomal S9 gene was carried out (Light Cycler 480; Roche) using GoTaq qPCR reagent (Promega), based on the synthesized cDNA. The primers used were 5'-TGCTGACGCTTGATGAGAAG-3' and 5'-CGCAGAGAAGTCGATGTG-3' for the ribosomal S9 gene as a housekeeping factor, and 5'-CCACCGAGGGACATACCGAT-3' and 5'-GTGCAAGGCTGGTGTTCTCA-3' for the NKp46 gene, with standardization by dividing the expression level of the NKp46 gene by the expression level of the ribosomal S9 gene. The significance of the results was determined by statistical testing using a multiple comparison test (Tukey) with SPSS statistics software.

### 3. Results

The Hibiscus flower fermentation extract promoted expression of the activation marker (NKp46) on the primary cultured human NK cells, at a concentration of 0.01 (v/v)% or greater (N = 4, *p < 0.001) (Fig. 2).

## Claims

1. An immune cell activator comprising a fermentation product of hibiscus or extract thereof as an active ingredient.

2. The immune cell activator according to claim 1, wherein the immune cells are NK cells.

3. The immune cell activator according to claim 1 or 2, wherein senescent cells are eliminated by activation of the immune cells.

4. The immune cell activator according to any one of claims 1 to 3, wherein the fermentation product of hibiscus or extract thereof is obtained by lactic acid bacteria fermentation of the hibiscus or extract thereof.

5. An anti-aging agent comprising a fermentation product of hibiscus or extract thereof as an active ingredient, **characterized in that** the anti-aging agent eliminates senescent cells by activation of immune cells.

6. The anti-aging agent according to claim 5, wherein the immune cells are NK cells.

7. The anti-aging agent according to claim 5 or 6, wherein the fermentation product of hibiscus or extract thereof is obtained by lactic acid bacteria fermentation of the hibiscus or extract thereof.

8. A method for activating immune cells for a subject in need thereof, comprising:
applying a fermentation product of hibiscus or extract thereof to the subject to activate the immune cells.

9. The method according to claim 8, wherein the immune cells are NK cells.

10. The method according to claim 8 or 9, wherein senescent cells are eliminated by activation of the immune cells.

11. The method according to any one of claims 8 to 10, wherein the fermentation product of hibiscus or extract thereof is obtained by lactic acid bacteria fermentation of the hibiscus or extract thereof.

12. A method for preventing or protecting against aging for a subject in need thereof, comprising:
applying a fermentation product of hibiscus or extract thereof to the subject to eliminate senescent cells by activation of immune cells.

13. The method according to claim 12, wherein the immune cells are NK cells.

14. The method according to claim 12 or 13, wherein the fermentation product of hibiscus or extract thereof is obtained by lactic acid bacteria fermentation of the hibiscus or extract thereof.
